# EUROPEAN PATENT APPLICATION

(11) **EP 4 696 724 A1**
(43) Date of publication of application: **18.02.2026**
(21) Application number: 24788631.0
(22) Date of filing: 03.04.2024
(51) Int. Cl.: C08F 220/10, A61F 2/16

(54) **SILICONE MONOMER MIXTURE AND SILICONE MONOMER COMPOSITION**

(30) Priority: 10.04.2023 JP 2023063206
(71) Applicant: Toray Industries, Inc., Tokyo 103-8666 (JP)
(72) Inventor: KITAGAWA, Rumiko, Otsu-shi, Shiga 520-8558 (JP)
(74) Representative: Hoefer & Partner Patentanwälte mbB
(86) International application number: PCT/JP2024/013707
(87) International publication number: WO 2024/214604

(57) **Abstract**

An object of the present invention is to provide a silicone monomer mixture having extremely good solubility in various solvents and other raw materials, and can realize a medical device having extremely high oxygen permeability and transparency. The present invention provides a silicone monomer mixture including a compound having a specific structure (I) and a compound having another specific structure (II), wherein in the mixture, the percentage of the compound of the specific structure (I) is 90.0 to 99.5%, the percentage of the compound having the other specific structure (II) relative to the total amount of the compound having the specific structure (I) and the compound having the other specific structure (II) is 0.01 to 3.0%.

## Description

### Technical Field

The present invention relates to a silicone monomer mixture and a silicone monomer composition.

### Background Art

Medical devices produced from silicone monomers as raw materials are widely used in wound dressings, ophthalmic lenses, and other applications due to their high oxygen permeability.

To improve the oxygen permeability and biocompatibility of medical devices molded using silicone monomers as raw materials, or to further increase their transparency, it is desirable for the silicone monomer to have a high purity as well as high compatibility with various solvents and other raw materials in the production process of the medical device.

One possible means of increasing the compatibility of the silicone monomer with various solvents and other raw materials, i.e., increasing the solubility of the silicone monomer itself, is to use the silicone monomer having a hydroxyl group that is hydrophilic.

(Mono-(2-hydroxy-3-methacryloxypropyl)-propyl ether-terminated polydimethyl siloxane is disclosed as an example of the silicone monomer having a hydroxyl group (Patent Literatures 1 and 2).

### Citation List

### Patent Literature

Patent Literature 1: JP 2015-500512 W
Patent Literature 2: JP 2021-515762 W

### Summary of Invention

### Technical Problem

However, other than the use of the silicone monomer having a hydroxyl group, there has hitherto been no means known to significantly enhance the solubility of the silicone monomer itself.

Therefore, an object of the present invention is to provide a silicone monomer mixture and a silicone monomer composition having extremely good solubility in various solvents and other raw materials, and can realize a medical device having extremely high oxygen permeability and transparency.

### Solution to Problem

In order to achieve the above object, the present invention provides a silicone monomer mixture and a silicone monomer composition, including a compound of the following general formula (I) and a compound of the following general formula (II): 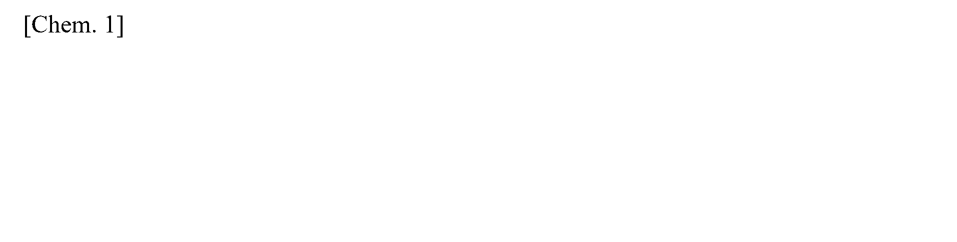 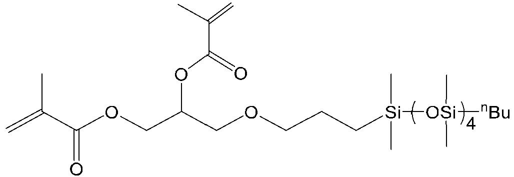 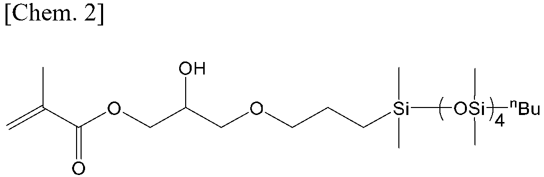 wherein the percentage of the compound of the general formula (I) in the mixture is 90.0 to 99.5%, and the percentage of the compound of the general formula (II) is 0.01 to 3.0% relative to the total amount of the compound of the general formula (I) and the compound of the general formula (II).

### Advantageous Effects of Invention

The present invention can provide a silicone monomer mixture, which has a very high solubility in various solvents and other raw materials although it does not have a hydroxyl group. As a result, a medical device having extremely high oxygen permeability and transparency can be achieved.

### Description of Embodiments

The silicone monomer mixture of the present invention includes a compound of the general formula (I) and a compound of the general formula (II), wherein in the mixture, the percentage of the compound of the general formula (I) is 90.0 to 99.5% by mass, and the percentage of the compound of the general formula (II) is 0.01 to 3.0% by mass relative to the total amount of the compound of the general formula (I) and the compound of the general formula (II).

In the present specification, the silicone monomer mixture refers to a mixture including the compound of the general formula (I) and the compound of the general formula (II).

As mentioned above, in the prior art, as a means of increasing the solubility of the silicone monomer, the silicone monomer having a hydroxyl group which is hydrophilic has been used. The present inventor has found that the compound with a specific structure of the general formula (I), having two methacrylic groups in a predetermined positional relationship, has extremely high solubility in various solvents and other raw materials, although it does not have a hydroxyl group, and has conceived the present invention. On the other hand, it is inevitable that the compound of the general formula (I) includes the compound of the general formula (II) as a so-called impurity. However, by keeping the percentage of the compound of the general formula (II) in the mixture within a certain range and increasing the purity of the compound of the general formula (I), it is possible to obtain a silicone monomer mixture which has extremely high solubility in various solvents and other raw material and can be preferably used as a raw material for a medical device.

The silicone monomer mixture of the present invention may be a mixture consisting only of the compound of the general formula (I) and the compound of the general formula (II), or a mixture including, in addition to the compound of the general formula (I) and the compound of the general formula (II), other silicone monomers or a compound other than the silicone monomer.

An example of the silicone monomer other than the compound of the general formula (I) and the compound of the general formula (II) may be a silicone monomer having a hydroxyl group which is hydrophilic, from the viewpoint of maintaining the solubility. Examples of the silicone monomer having a hydroxyl group include (mono-(2-hydroxy-3-methacryloxypropyl)-propyl ether-terminated polydimethyl siloxane and N-(3-(3-(9-butyl-1,1,3,3,5,5,7,7,9,9-decamethylpentasiloxanyl)propoxy)-2-hydroxypropyl)acrylamide).

An example of the compound other than the silicone monomer may be methacrylic anhydride, or a decomposition product or a methacrylate of a polymerization inhibitor.

The structure of the compound, other than the silicone monomer, which is included in the silicone monomer mixture, can be analyzed by high-resolution GC/MS measurements. Details of the analytical method are described below.

The percentage of the compound of the general formula (I) in the silicone monomer mixture of the present invention is preferably 93.0 to 99.0%, and more preferably 95.0 to 98.0% from the viewpoint of increasing the efficiency of the production process such as purification, while a raw material for a medical device with high transparency is obtained. The percentage of the compound of the general formula (I) used herein refers to a ratio of an area of a peak of the compound of the general formula (I) to an area of entire detection peak obtained by GC/FID measurement of the silicone monomer mixture. Details of the analytical method are described below.

In the silicone monomer mixture, the percentage of the compound of the general formula (II) is preferably 0.02 to 2.5%, and more preferably 0.04 to 2.0%, relative to the total amount of the compound of the general formula (I) and the compound of the general formula (II), because no additional purification step is required.

The percentage of the compound of the general formula (II) used herein refers to a ratio of the peak area of the compound of the general formula (II) to the total peak area of the peak of the compound of the general formula (I) and the peak of the compound of the general formula (II) in the detection peak obtained by GC/FID measurement of the silicone monomer mixture.

The silicone monomer mixture of the present invention can be suitably used as the silicone monomer composition composed of the silicone monomer mixture and 2-hydroxyethyl methacrylate. In the present invention, the silicone monomer composition refers to a composition including the silicone monomer and 2-hydroxyethyl methacrylate.

2-Hydroxyethyl methacrylate is heavily used as a monomer to produce medical devices such as ophthalmic lenses. In the silicone monomer composition of the present invention, the compound of the general formula (I), which is the main component of the silicone monomer mixture of the present invention, is easily dissolved in 2-hydroxyethyl methacrylate or other raw material which is hydrophilic, although the compound does not have a hydroxyl group which is hydrophilic. Thus, for example, even when the silicone monomer mixture of the present invention is included in the silicone monomer composition at a ratio as high as 50 % by mass, it is possible to provide a raw material of a medical device with high transparency.

The percentage of the silicone monomer mixture in the silicone monomer composition composed of the silicone monomer mixture and 2-hydroxyethyl methacrylate is preferably 0.001 to 99 % by mass, more preferably 0.010 to 80 % by mass, and even more preferably 0.100 to 50 % by mass, since the raw material is more soluble in other raw material and the raw material for the medical device with a higher transparency can be obtained.

The percentage of 2-hydroxyethyl methacrylate in the composition is preferably 1 to 99 % by mass, more preferably 5 to 70 % by mass, and even more preferably 10 to 50 % by mass, since the raw material for the medical device with a higher transparency can be obtained.

Furthermore, the silicone monomer composition of the present invention may be a composition consisting only of the silicone monomer mixture and 2-hydroxyethyl methacrylate, or it may include other hydrophilic compounds.

When the other hydrophilic compounds are included, the percentage of the other hydrophilic compounds in the composition is preferably 1 to 99 % by mass, more preferably 5 to 70 % by mass, and even more preferably 10 to 50 % by mass, since the raw material for the medical device with a higher transparency can be obtained.

Examples of "the other hydrophilic compounds" include compounds having a structure containing an unsaturated group, such as vinyl amide, vinyl imide, vinyl lactam, hydrophilic (meth)acrylate, (meth)acrylamide and derivatives thereof, the hydrophilic styrene-based compounds, vinyl ether, vinyl carbonate, vinyl carbamate, and vinyl urea.

More specific examples of the compound that retains transparency of the raw material for producing a medical device as the other hydrophilic compound include N,N-dimethylacrylamide, N-vinylpyrrolidone, N-vinyl-2-piperidone, N-vinyl-2-caprolactam, N-vinyl-3-methyl-2-caprolactam, N-vinyl-3-methyl-2-piperidone, N-vinyl-4-methyl-2-piperidone, N-vinyl-4-methyl-2-caprolactam, N-vinyl-3-ethyl-2-pyrrolidone, N-vinyl-4,5-dimethyl-2-pyrrolidone, vinylimidazole, N,N-diethylacrylamide, acrylamide, N,N-bis(2-hydroxyethyl)acrylamide, acrylonitrile, N-isopropylacrylamide, 2-ethyloxazoline, N-(2-hydroxypropyl)(meth)acrylamide, N-(2-hydroxyethyl)(meth)acrylamide, 2-methacryloyloxyethyl phosphorylcholine, 3-(dimethyl(4-vinylbenzyl)ammonio)propane-1-sulfonate (DMVBAPS), 3-((3-acrylamidopropyl)dimethylammonio)propane-1-sulfonate (AMPDAPS), 3-((3-methacrylamidopropyl)dimethylammonio)propane-1-sulfonate (MAMPDAPS), 3-((3-(acryloyloxy)propyl)dimethylammonio)propane-1-sulfonate (APDAPS), 3-((3-methacryloyloxy)propyl)dimethylammonio)propane-1-sulfonate (MAPDAPS), N-vinyl-N-methylacetamide, N-vinylacetamide, N-vinyl-N-methylpropionamide, N-vinyl-N-methyl-2-methylpropionamide, N-vinyl-2-methylpropionamide, N-vinyl-N,N'-dimethylurea, dimethylaminopropyl (meth)acrylamide, dimethylaminoethyl (meth)acrylamide, dimethylaminopropyl (meth)acrylate and dimethylaminoethyl (meth)acrylate and methoxy polyethylene glycol (meth)acrylate.

Among them, N,N-dimethylacrylamide, N-vinylpyrrolidone, N-vinyl-2-piperidone, N-vinyl-2-caprolactam, N-vinyl-3-methyl-2-caprolactam, N-vinyl-3-methyl-2-piperidone, N-vinyl-4-methyl-2-piperidone, N-vinyl-4-methyl-2-caprolactam, N-vinyl-3-ethyl-2-pyrrolidone, N-vinyl-4,5-dimethyl-2-pyrrolidone, vinylimidazole, N,N-diethylacrylamide, N-isopropylacrylamide, N-(2-hydroxypropyl)(meth)acrylamide, N-vinyl-N-methylacetamide, N-vinylacetamide, N-vinyl-N-methylpropionamide, N-vinyl-N-methyl-2-methylpropionamide, N-vinyl-2-methylpropionamide, dimethylaminopropyl (meth)acrylamide, dimethylaminoethyl (meth)acrylamide, dimethylaminopropyl(meth)acrylate or dimethylaminoethyl (meth)acrylate or methoxypolyethyleneglycol (meth)acrylate is preferred, N,N-dimethylacrylamide, N-vinylpyrrolidone, N-vinyl-N-methylacetamide or methoxy polyethylene glycol (meth)acrylate is more preferred, and N,N-dimethylacrylamide is still more preferred.

When producing the silicone monomer composition from the silicone monomer mixture, it is also preferable to add an additional silicone monomer (e.g., a compound of the general formula (II)) having a hydroxyl group in addition to that contained in the silicone monomer mixture.

The silicone monomer mixture and silicone monomer composition of the present invention are preferably used as raw materials for producing medical devices.

Examples of the medical device include an ophthalmic lens, a dermal covering material, a wound dressing material, a skin protection material, a skin medicine carrier, an infusion tube, a gas delivery tube, a drainage tube, a blood circuit, a covering tube, a catheter, a stent, a sheath, a biosensor chip, an artificial heart and lung, or an endoscopic covering material. In particular, they can be suitably used as the raw materials for the ophthalmic lens, as they can enhance the transparency in addition to exhibiting a sufficient oxygen permeability. Here, examples of the ophthalmic lens include a contact lens, an intraocular lens, an artificial cornea, a corneal inlay, a corneal onlay, and an eyeglass lens.

### Examples

The present invention will now be described more specifically by way of examples, but the present invention is not limited to these Examples. First, analytical methods and evaluation methods will be shown below.

### <Structural Analysis of Silicone Monomer Mixture>

The structure of the compound contained in the silicone monomer mixture was analyzed by performing a high-resolution GC/MS measurement using a measurement sample prepared by dissolving 100 mg of the silicone monomer mixture in 0.5 mL of hexane to 20% (w/v). The measurement conditions are shown below.
GC: 7890B (Agilent)
Column: SH-Rxi-5Sil (30m × 0.25 mm id; film thickness, 1.0 µm)
Temperature rising condition: 50°C (1 min) - 10 °C/min → 300°C (14 min)
Column flow rate: He 1 mL/min (Constant Flow)
Inlet temperature: 250°C
Injection amount: 1 µL (EI50: 1 split, CI20: 1 split)
MS: JMS-T100GCV (JEOL)
Ionization method: EI+, CI+
CI gas: Isobutane
Ion source temperature: EI 250°C, CI 200°C
Resolution capability: EI > 8000 (at m/z 264), CI > 8000 (at m/z 264)
Range of mass measurement: EI m/z 20 - 800, CI m/z 60 - 1000.

### <Percentages of Compound of General Formula (I), Compound of General Formula (II), and Other Compounds in Silicone Monomer Mixture>

0.05 g of the silicone monomer mixture was weighed into a measuring flask (10 mL) and the volume was adjusted to a predetermined amount with toluene, which was used as a measurement sample to perform GC/FID measurement. The measurement conditions for GC/FID are shown below.

The ratio of the peak area of the compound of the general formula (I) to the area of the entire obtained detection peak was determined as the percentage of the compound of the general formula (I) relative to the silicone monomer mixture. Similarly, the ratio of the peak area of the compound of the general formula (II) to the total peak area of the obtained detection peak was determined as the percentage of the compound of the general formula (II) relative to the silicone monomer mixture. When the compound other than the compound of the general formula (I) and the compound of the general formula (II) were included, the percentages of those compounds were calculated in the same manner.

The thus obtained ratio of the peak area of the general formula (II) to the total peak area of the compound of the general formula (I) and the compound of the general formula (II) was determined as the percentage of the compound of the general formula (II) to the total volume of the compound of the general formula (I) and the compound of the general formula (II).
Equipment: GC6890 (Agilent technologies)
Column: SH-Rxi-5Sil MS (RESTEK)
Column temperature: 50°C (1 min) - 10 °C/min - 300°C (10 min)
Inlet gas pressure: 1.82 mL/min (Helium, constant flow rate mode)
Split ratio: 1:10
Inlet temperature: 250°C
Detector temperature: 300°C
Injection amount: 1 µL.

### <Evaluation of Solubility of Silicone Monomer Mixture or Silicone Monomer Based on Visual Evaluation>

10 g of the silicone monomer composition placed in a glass screw tube was visually observed to confirm the solubility of the silicone monomer mixture and the silicone monomer in the composition.

### <Evaluation of Solubility of Silicone Monomer Mixture or Silicone Monomer Based on Total Light Transmittance>

Using an SM color computer (model SM-7-CH; manufactured by Suga Test Instruments Co., Ltd.), 6 g of the silicone monomer composition placed in a glass screw tube was measured. The acceptance criteria for the transparency is to be a total light transmittance of 80% or more.

### [Examples 1 to 4]

The analysis described above was performed for four types of the silicone monomer mixture (manufactured by Toray Industries, Inc.), including the compound of the general formula (I). The results of the analysis show that the percentage of the compound of the general formula (I) and the compound of the general formula (II) contained in the sample, the percentage (%) of the compound of the general formula (II) relative to the total amount of the compound of the general formula (I) and the compound of the general formula (II), the percentage (%) of all compounds other than the compound of the general formula (I) and the compound of the general formula (II), and the most abundant structure other than the compound of the general formula (I) are as shown in Table 1.

### [Comparative Example 1]

The same analysis as in Example 1 was performed on the silicone monomer FM0711 (manufactured by JNC CORPORATION; average molecular weight announced by the manufacturer, 1,000), which has one methacrylic group and no hydroxyl group. The results are as shown in Table 1.

### [Comparative Example 2]

The same analysis as in Example 1 was performed on the silicone monomer FM0721 (manufactured by JNC Corporation; average molecular weight announced by the manufacturer, 5,000), which has one methacrylic group and no hydroxyl group. The results are as shown in Table 1.

### [Comparative Example 3]

The same analysis as in Example 1 was performed on the silicone monomer FM0725 (manufactured by JNC CORPORATION; average molecular weight announced by the manufacturer, 10,000), which has one methacrylic group and no hydroxyl group. The results are as shown in Table 1.

### [Comparative Example 4]

The same analysis as in Example 1 was performed on the silicone monomer FM0726 (manufactured by JNC CORPORATION; average molecular weight announced by the manufacturer, 21,500), which has two methacrylic groups and no hydroxyl group. The results are as shown in Table 1.

### [Table 1]

**[Table 1]**

| | Percentage of compound of general formula (I) (%) | Percentage of compound of general formula (II) (%) | Percentage of compound of general formula (II) relative to total amount of compound of general formula (I) and compound of general formula (II) (%) | Percentage of all compounds other than compound of general formula (I) and compound of general formula (II) (%) | Structure of most abundant compound other than compound of general formula (I) |
|---|---|---|---|---|---|
| Example 1 | 97.37 | 0.36 | 0.37 | 2.27 | 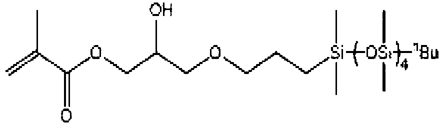 |
| Example 2 | 97.31 | 0.32 | 0.33 | 2.37 | 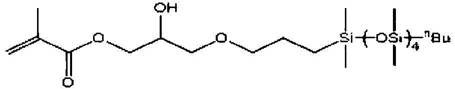 |
| Example 3 | 90.00 | 1.50 | 1.64 | 8.50 | 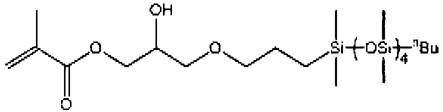 |
| Example 4 | 95.00 | 0.50 | 0.52 | 4.50 | 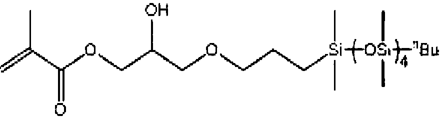 |
| Comparative Example 1 | 0 | 0 | 0 | 100 | 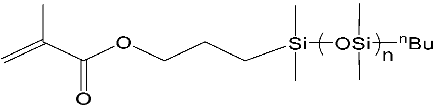 |
| Comparative Example 2 | 0 | 0 | 0 | 100 | 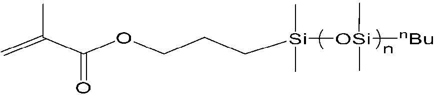 |
| Comparative Example 3 | 0 | 0 | 0 | 100 | 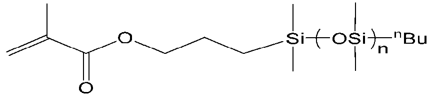 |
| Comparative Example 4 | 0 | 0 | 0 | 100 | 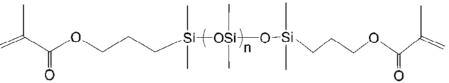 |

### <Preparation of Silicone Monomer Composition>

Each component of the silicone monomer composition was collected in a glass screw tube at the percentages shown in Table 2, taking the total weight as 10 g. The silicone monomer composition was prepared by shaking the screw tube by hand 20 times at room temperature (20 to 23°C).

### <Transparency of Silicone Monomer Composition>

The visual evaluation and total light transmittance evaluation were performed on the silicone monomer compositions 1 to 15. The evaluation results are as shown in Table 2. When the silicone monomer mixtures of Examples 1 to 4 were used, no turbidity or separation was observed in the compositions upon visual evaluation, and the total light transmittance, an indicator of the transparency, was sufficiently high and good.

### [Table 2]

**[Table 2]**

| Composition | Silicone monomer mixture or silicone monomer | Content of silicone monomer mixture or silicone monomer (% by mass) | Content of 2-hyroxyethyl methacrylate (% by mass) | Content of compound of general formula (II) (% by mass)*1 | Visual evaluation | Total light transmittance (%) |
|---|---|---|---|---|---|---|
| 1 | Example 1 | 50 | 50 | 0 | Soluble (Colorless and transparent) | 95 |
| 2 | Example 1 | 99 | 1 | 0 | Soluble (Colorless and transparent) | 97 |
| 3 | Example 2 | 50 | 25 | 25 | Soluble (Colorless and transparent) | 96 |
| 4 | Example 2 | 95 | 2.5 | 2.5 | Soluble (Colorless and transparent) | 96 |
| 5 | Example 2 | 50 | 50 | 0 | Soluble (Colorless and transparent) | 97 |
| 6 | Example 3 | 50 | 50 | 0 | Soluble (Colorless and transparent) | 95 |
| 7 | Example 4 | 50 | 50 | 0 | Soluble (Colorless and transparent) | 97 |
| 8 | Comparative Example 1 | 50 | 50 | 0 | Insoluble (Separated into two layers) | 51 |
| 9 | Comparative Example 2 | 50 | 50 | 0 | Insoluble (Separated into two layers) | 49 |
| 10 | Comparative Example 3 | 50 | 50 | 0 | Insoluble (Separated into two layers) | 53 |
| 11 | Comparative Example 4 | 50 | 50 | 0 | Insoluble (Separated into two layers) | 45 |
| 12 | Comparative Example 2 | 5 | 47.5 | 47.5 | Insoluble (Turbidness) | 50 |
| 13 | Comparative Example 2 | 95 | 2.5 | 2.5 | Insoluble (Turbidness) | 39 |
| 14 | Comparative Example 3 | 5 | 47.5 | 47.5 | Insoluble (Turbidness) | 30 |
| 15 | Comparative Example 3 | 95 | 2.5 | 2.5 | Insoluble (Turbidness) | 35 |

| | | | | | | |
|---|---|---|---|---|---|---|
| *1 excluding compound of general formula (II) in silicone monomer mixture | | | | | | |

In addition, the "content of the compound of the general formula (II)" in footnote 1 of Table 2 refers to the amount of the compound of the general formula (II) added in addition to that contained in the silicone monomer mixtures of Examples 1 to 4 when producing the silicone monomer mixture.

## Claims

1. A silicone monomer mixture comprising:
a compound of the following general formula (I); and
a compound of the following general formula (II): 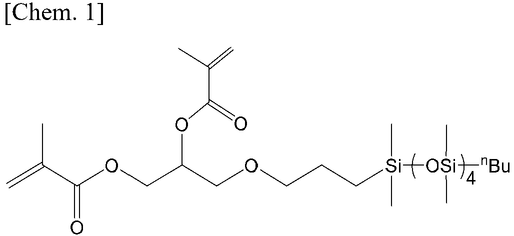 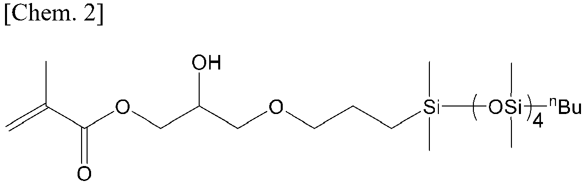
wherein the percentage of the compound of the general formula (I) in the mixture is 90.0 to 99.5%, and
the percentage of the compound of the general formula (II) is 0.01 to 3.0% relative to the total amount of the compound of the general formula (I) and the compound of the general formula (II).

2. A silicone monomer composition composed of the silicone monomer mixture according to claim 1 and 2-hydroxyethyl methacrylate.

3. The silicone monomer mixture according to claim 1, wherein the silicone monomer mixture is used for production of a medical device.

4. The silicone monomer mixture according to claim 3, wherein the medical device is an ophthalmic lens.

5. The silicone monomer composition according to claim 2, wherein the silicone monomer composition is used for production of a medical device.

6. The silicone monomer composition according to claim 5, wherein the medical device is an ophthalmic lens.
